# EUROPEAN PATENT APPLICATION

(11) **EP 3 150 167 A1**
(43) Date of publication of application: **05.04.2017**
(21) Application number: 16275145.7
(22) Date of filing: 30.09.2016
(51) Int. Cl.: A61F 2/07

(54) **MODULAR SYSTEM WITH AT LEAST ONE BRANCH GRAFT**

(30) Priority: 30.09.2015 US 201514871394
(71) Applicant: Cook Medical Technologies LLC, Bloomington, IN 47404 (US)
(72) Inventor: ROEDER, Blayne A., Bloomington, IN 47401 (US)
(74) Representative: Williams Powell

(57) **Abstract**

A modular system for placement in a patient is disclosed. In one exemplary embodiment, a first tubular body portion (12) and a second tubular body portion (14) are provided. The second tubular body portion (14) may be located at least partially distally of the first body portion (12). A distal end of the first tubular body portion (12) may comprise a first taper, and a proximal end of the second tubular body portion (14) may comprise a second taper. The first and second taper may form a working space in a deployed state.

## Description

The present invention relates to modular systems and methods therefor.

### BACKGROUND

In recent years endovascular implantable devices have been developed for treatment of aortic aneurysms. These devices are delivered to the treatment site through the vascular system of the patient rather than by open surgery. The devices include a tubular or cylindrical framework or scaffolding of one or more stents to which is secured a tubular shape of graft material such as woven Dacron, polyester polytetrafluoroethylene, or the like. The devices are initially reduced to a small diameter and placed into the leading or proximal end of a catheter delivery system whereafter the delivery system is inserted into the vascular system of the patient such as through a femoral incision. The leading end of the delivery system is maneuvered to the treatment site over a previously positioned guide wire. Through manipulation of a control system that extends to the proximal end of the catheter from the distal end of the system outside the patient, the implantable device can be deployed by holding the device at its location and withdrawing a surrounding sheath. The implantable device or stent graft can then self-expand or be expanded through the use of a balloon which is introduced with a stent graft introduction device. The stent graft becomes anchored into position in healthy wall tissue of the aorta, by barbs for example. The delivery system is then removed leaving the expandable device in position to treat an aneurysm in the aorta in a manner that channels all blood flow through the stent graft so that little to no blood flow enters the aneurysm. As a result, not only does the aneurysm no longer continue to grow and possibly rupture but the aneurysm actually begins to shrink and commonly disappears entirely.

For treatment of thoracic aortic aneurysms in particular it is necessary to introduce the implantable device high up in the aorta and in a region of the aorta which is curved and where there can be strong blood flow.

In the thoracic aorta there are major branch vessels, the brachiocephalic, the left carotid, and the left subclavian. For treatment of an aneurysm in the region of the thoracic arch provision must be made for blood supply to continue to these arteries. For this purpose fenestrations may be provided into the wall of a stent graft in that region. Access is generally obtained to these fenestrations, to deploy side arms into the stent graft, via the left or right brachial arteries or less commonly via the left or right carotid arteries. Once into the thoracic arch via such an artery the fenestration in the stent graft must be catheterized. In some cases, it may be difficult to assemble one or more stent grafts in the thoracic aorta, particularly due to anatomical considerations involved.

### BRIEF SUMMARY

The present invention seeks to provide an improved modular system and method therefor.

According to an aspect of the invention, there is provided a modular system as in claim 1.

Some embodiments include features according to one or more of dependent claims 2 to 8.

In some embodiments, the first tubular body portion at least partially overlaps with the second tubular body portion.

In some embodiments, a proximal end of the second tubular body portion overlaps a distal end of the first tubular body portion.

Some embodiments comprise a third tubular body portion located at least partially distally of the second tubular body portion.

In some embodiments, the first branch graft is configured to be deployed through the working space.

According to an aspect of the invention, there is provided a modular system as in claim 9.

Some embodiments include features according to one or more of dependent claims 10 to 15.

According to an aspect of the invention, there is provided a method for deploying a modular system, the method comprising: providing a first tubular body portion comprising a first taper and a second tubular body portion comprising a second taper; deploying the second tubular body portion at least partially distally of the first body portion, wherein the first body portion and the second body portion are engaged at a junction; forming a working space adjacent the first and second tapers in a deployed state; deploying a proximal branch graft such that it is coupled to the first tubular body portion and extends through a first portion of the working space; and deploying a distal branch graft such that it is coupled to the second tubular body portion and extends through a second portion of the working space.

The method can be according to the above, wherein the proximal branch graft comprises a first opening facing proximally within the first tubular body portion, and wherein the distal branch graft comprises a second opening facing distally within the second tubular body portion.

The method can be according to the above, further comprising: providing a third tubular body portion located distally of the second tubular body portion; and coupling the third tubular body portion to the second tubular body portion at a second junction.

The method can be according to the above, wherein the proximal branch graft is a first branch graft, wherein a second branch graft is coupled to one of the first tubular body portion or the second tubular body portion, and wherein the distal branch graft is a third branch graft.

The method can be according to the above, wherein the second branch graft is coupled to the first tubular body portion and extends through the first portion of the working space in the deployed state.

A modular system for placement in a patient is disclosed. In one exemplary embodiment, a first tubular body portion and a second tubular body portion are provided. The second tubular body portion may be located at least partially distally of the first body portion. A distal end of the first tubular body portion may comprise a first taper, and a proximal end of the second tubular body portion may comprise a second taper. The first and second taper may form a working space in a deployed state.

### BRIEF DESCRIPTION OF THE DRAWINGS

Embodiments of the invention are described below, by way of example only, with reference to the accompanying drawings.
FIG. 1 is a front illustration of a modular arch system.
FIG. 2 is a front illustration of a modular arch system located in the thoracic arch of a patient.
FIG. 3 is a front illustration of a portion of a thoracic arch of a patent prior to the deployment of a modular arch system.
FIG. 4 is an illustration showing the deployment of a second tubular body portion of a modular arch system within the thoracic arch of a patient.
FIG. 5 is an illustration showing the deployment of a first tubular body portion and a second tubular body portion of a modular arch system within the thoracic arch of a patient.
FIGS. 6A-C are three illustrations showing three non-limiting embodiments of a structure for providing communication between a tubular body and a branch graft.

### DETAILED DESCRIPTION OF THE DRAWINGS AND THE PRESENTLY PREFERRED EMBODIMENTS

Throughout this specification the term "distal" with respect to a portion of the aorta, a deployment device or a prosthesis such as a stent graft is intended to mean the end of the aorta, deployment device, or prosthesis further away in the direction of blood flow from the heart and the term proximal is intended to mean the portion of the aorta, deployment device, or end of the prosthesis nearer to the heart.

Throughout this discussion the term "stent graft" is intended to mean a device which has a generally tubular body of biocompatible graft material and at least one stent coupled to the tubular body to define a lumen through the stent graft. The stent graft may be bifurcated and have fenestrations, side arms or the like. Other arrangements of stent grafts may be used.

Exemplary stent grafts disclosed herein may comprise Dacron. The generally tubular body may, for example, comprise a plurality of zig-zag stents for providing support during the operation of the device. Further, certain embodiments may comprise branch stent grafts connected to, and branching from, the generally tubular body. Exemplary stent graft embodiments, including embodiments with branch stent grafts and zig-zag stents, are shown in U.S. Patent Appl. Pub. No. 2011-0257731 A1 to Hartley et al., and U.S. Patent 8,021,419 to Hartley et al., each of which is incorporated herein in its entirety.

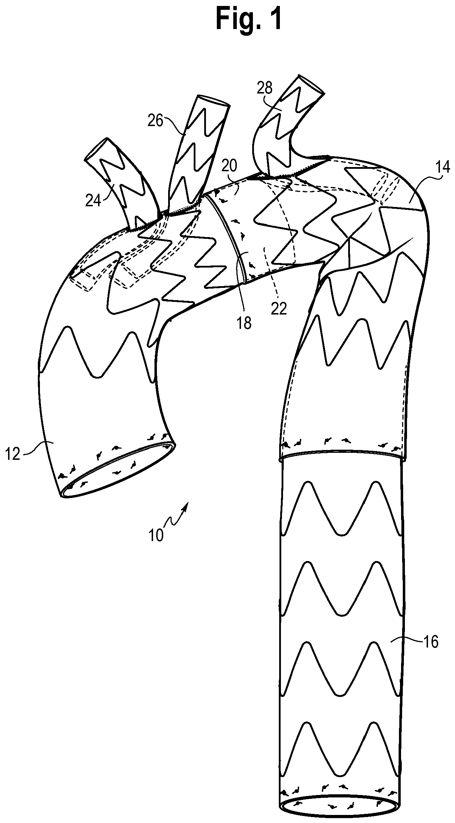

An exemplary modular system may comprise a stent graft assembly with a generally tubular body comprising a lumen therethrough and with one or more branch stent grafts branching therefrom. Referring to FIG. 1, stent graft assembly 10 may, for example, be configured for placement in a thoracic arch of a patient. Stent graft assembly 10 may comprise a first tubular body portion 12 configured for at least partial placement in the ascending aorta, and a second tubular body portion 14 located at least partially distally of first tubular body portion 12. In some embodiments, a proximal end 20 of second tubular body portion 14 may engage with a distal end 22 of first tubular body portion 12 at a junction 18. For example, as depicted, proximal end 20 overlaps and/or engages with distal end 22 by frictional engagement within distal end 22. First tubular body portion 12 and second tubular body portion 14 are preferably configured to engage in a manner as to provide a sufficient seal at junction 18 to prevent a substantial amount of fluid from leaking from internally to externally of stent graft assembly 10. In some embodiments, including the embodiment depicted by FIG. 1, stent graft assembly 10 comprises a third tubular body portion 16 located at least partially distally of the second tubular body portion 14.

The diameter of the components of the stent graft assembly 10 may vary to properly fit the aorta of a patient. For example, the proximal end of first tubular body portion 12 may comprise a larger diameter than other areas of stent graft assembly 10 to correspond with a potentially larger portion of the aorta. The diameters of first tubular body portion 12, second tubular body portion 14, and third tubular body portion 14, as well as the diameters and sizes of other components, may vary in a way suitable to fit a diseased aorta.

Select components of the stent graft assembly 10 may further comprise one or more branch portions for facilitating the flow of fluid through branch vessels extending from the aorta. In the embodiment of FIG. 1, stent graft assembly 10 comprises first branch graft 24, second branch graft 26, and third branch graft 28. First branch graft 24, for example, may be configured for placement at least partially in the brachiocephalic artery of a patient. Similarly, second branch graft 26 may be configured for at least partial placement in the common or left carotid artery, and third branch graft 28 may be configured for at least partial placement in the left subclavian artery.

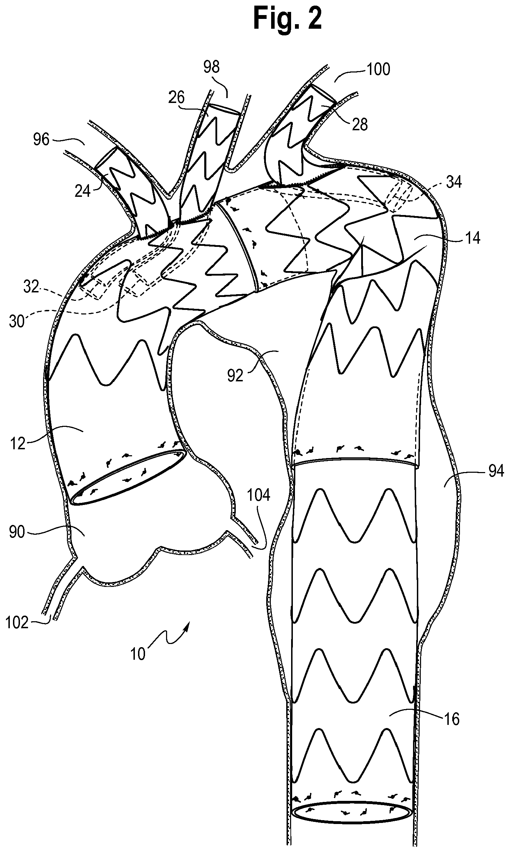

FIG. 2 shows the herein described embodiment of stent graft 10 deployed within a diseased thoracic arch 92 of a patient. Thoracic arch 92 is illustrated with an aneurysm. First tubular body portion 12 of stent graft assembly 10 communicates with ascending aorta 90. As shown, the proximal end of first tubular body portion 12 terminates before the coronary arteries 102 and 104 to prevent blockage of blood flow. However, in other embodiments, first tubular body portion 12 may extend to or proximally beyond the coronary arteries and be provided with corresponding fenestrations or branches to facilitate coronary circulation.

In the depicted embodiment, first branch graft 24 and second branch graft 26 extend from first tubular body portion 12 and are respectively configured to communicate with the brachiocephalic artery 96 and the common or left carotid artery 98. Branch grafts 24 and 26 comprise openings 30 and 32, which may face proximally such that the direction of flow through the openings 30 and 32 is the same as the direction of flow internally of stent graft assembly 10 adjacent to the openings.

Referring to FIG. 2, a third branch graft 28 may also be provided. In the depicted embodiment, third branch graft 28 extends from second tubular body portion 14 and is in communication with left subclavian artery 100. An opening 34 at the proximal end of third branch graft 28 faces in the distal direction, which may be opposite, or retrograde, to the direction of similar openings (i.e., openings 30 and 32) provided with first and second branch grafts 24 and 26. Accordingly, the direction of blood flow at the proximal end of third branch graft 28 will be opposite the direction of blood flow within the adjacent portion of stent graft assembly 10. Fluid pressure within stent graft assembly 10 will operate to force blood through opening 34 to provide sufficient blood flow to a branch vessel in communication with third branch graft 34. In other embodiments, third branch graft 34 may include a bend, helix, or another structure configured to orient opening 34 to face the proximal direction. In some other embodiments, a third branch graft may extend from first tubular body portion 12.

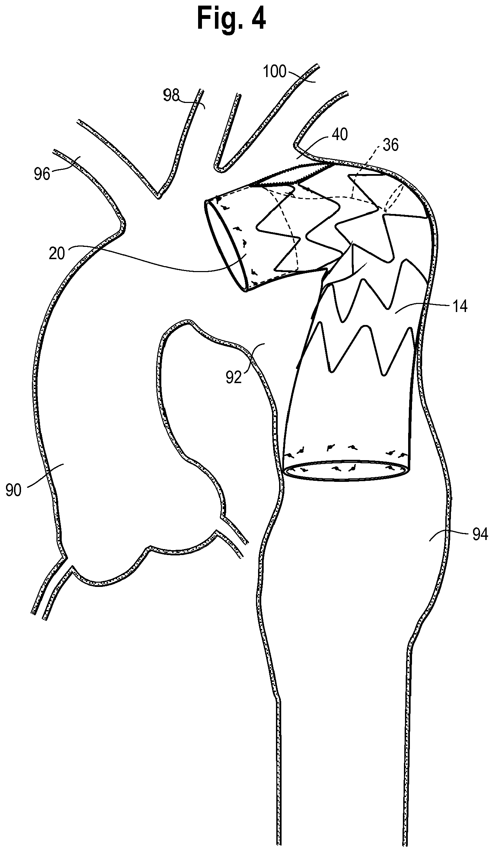

Advantageously, the current embodiment provides a working space near the upper wall of the thoracic arch. Referring to FIGS. 4-5, a space 40 may be provided between an upper wall of thoracic arch 92 and an outer surface of second tubular body portion 14. As depicted, the diameter of proximal end 20 comprises a taper or a diameter smaller than the diameter of other portions of second tubular body portion 14, thereby forming a gap or area defining the space 40 adjacent to proximal end 20. Space 40 may provide the room or the working space for the deployment of one or more branch vessels, including the necessary working space for the deployment of third branch graft 28.

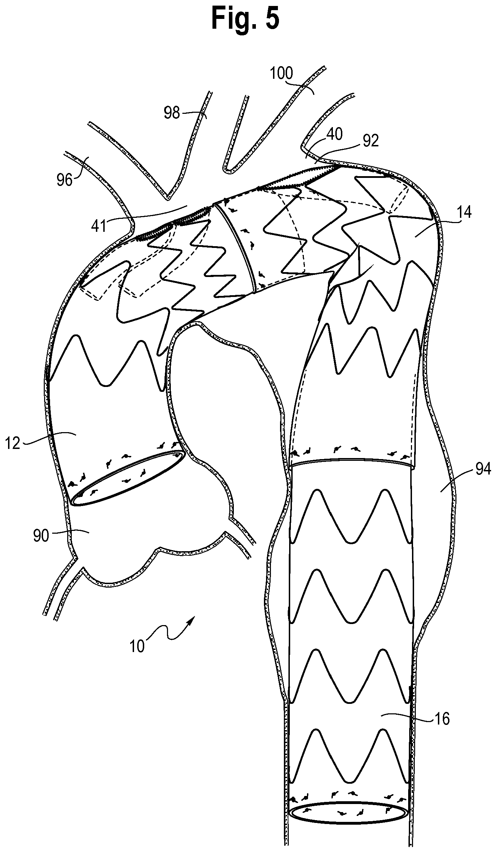

Similarly, as best seen in FIG. 5, first tubular body portion 12 may have a distal end 22 with a taper or diameter smaller than the diameter of other portions of first tubular body portion 12. This smaller diameter or tapered distal end 22 may be positioned such that, when deployed, a space 41 is provided between an upper wall of thoracic arch 92 and first tubular body portion 12. Spaces 40 and 41 (either together or individually) may combine to provide working space or room for the deployment of one or more branch grafts or other devices.

The described embodiments with spaces 40 and 41 also advantageously reduce confinement within stent graft assembly 10, thereby providing more room for fluids to flow and more working space for the deployment and installation of modular components. Further, the space-saving attributes may allow for the use of larger branch grafts without overly-restricting overall blood-flow through stent graft assembly 10, which may result in more blood flow both through the aorta and through the branch vessels.

It may be desired to cannulate the first and second branch grafts 24 and 26 from access sites located from above (and distally) for deployment in the proximal direction within their corresponding branch vessels. For example, first branch graft 24 and second branch graft 26 may be deployed through an access site located above (and distally) within branch vessels, such as the brachiocephalic artery 96 and the common or left carotid artery 98, respectively. However, it may be difficult to provide a third access site from above. Providing third branch graft 28 with opening 34 configured to face in the distal direction within stent graft assembly 10 may allow for an easier access site located below (and distally), such as in a lower location in the descending aorta 94. In other words, third branch graft 28 may move up the descending aorta 94 and into its operation position during installation. This may be most easily accomplished when opening 34 is configured to face in the distal direction during operation. In some embodiments, opening 34 may transition into an internal tube having a helical portion extending proximally to reduce the angle or bend of third branch graft 28 when deployed. This feature may operate to eliminate a sudden change in direction of particles flowing through third branch graft 28, which may conserve momentum and reduce friction forces. An internal helical conduit may also operate to prevent constriction or kinking of third branch graft 28.

Providing third branch graft 28 as described herein may also be advantageous over providing a fenestration corresponding to a third branch vessel (as described above), as a fenestration may limit the level of engagement between first tubular body portion 12 and second tubular body portion 14 (see FIGS. 1-2). In other words, the overlap zone or distance may be affected by the use of a fenestration, as the area adjacent to the entry of a third branch vessel is located nearly adjacent to junction 18. Third branch graft 28, on the other hand, may extend from a location of stent graft assembly 10 located distally of junction 18 as described herein.

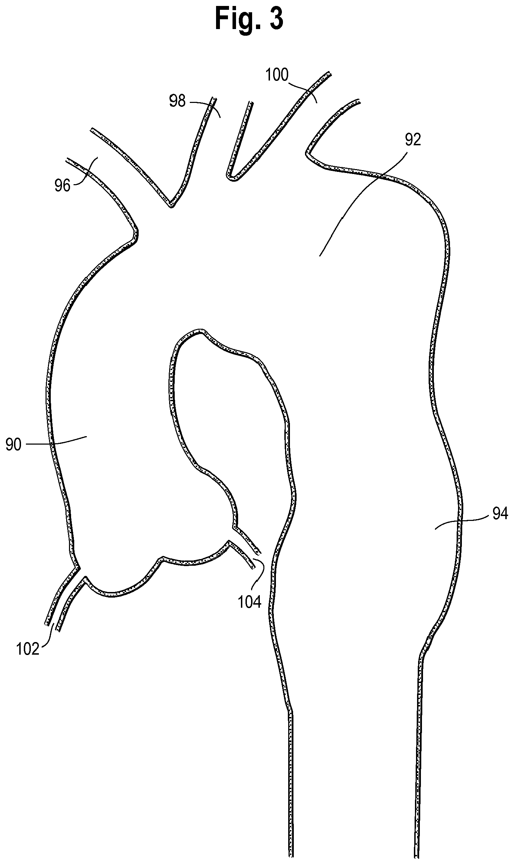

The disclosed embodiments may be installed in a modular manner. FIG. 3 shows a diseased thoracic arch 92 without prior to the installation of a stent graft. Referring to FIG. 4, second tubular body portion 14 may be deployed at least partially within thoracic arch 92 by itself and prior to the installation or deployment of other portions. Second tubular body portion 14 may, by non-limiting example, enter a patient's descending aorta 92 at a location distally of its depicted position (in FIG. 4), and then may be moved proximally within descending aorta 94 to its operational position as depicted. The second tubular body portion 14 may then be expanded as described herein, or, , the expansion may be delayed until the deployment and/or installation of other components.

Referring to FIG. 5, first tubular body portion 12 may be deployed or installed after second tubular body portion 14, although in other embodiments it may be deployed or installed prior to second tubular body portion 14. First tubular body portion 12 may be inserted into the aorta at an access location distally of its depicted position, and then may be moved proximally within descending aorta 94 and through thoracic arch 92 (and potentially through second portion 14) to its operational position. As previously described, a space 41 may be provided between an upper wall of the thoracic arch of the patient and an outer wall of tubular body portion 12 to facilitate the deployment of the branch grafts. In other embodiments the first tubular body portion 12 may enter the aorta through an access location in a branch vessel, such as through on of the brachiocephalic artery, common or left carotid artery, or left subclavian artery. First tubular body portion 12 may expand into its operational shape when its distal end is surrounded by the proximal end of second tubular body portion 14 such that the expansion provides contact and engagement. Similarly, third tubular body portion 16 may be deployed and installed at a position distal of second tubular body portion 14.

Next, referring to FIG. 2, the branch grafts 24, 26, and 28 may be installed. By non-liming example, the branch grafts may enter and proceed to their operational positions from the descending aorta 94 (thereby moving proximally to their operational positions) or from distally within their respective branch vessels. As described above, it may be desirable for first branch graft 24 and second branch graft 26 to enter through access locations of branch vessels 96 and 98, but for third branch graft 28 to enter through an access location located in the descending aorta 94. However, the branch grafts are not limited to deployment through any particular access location. Further, one or more of the branch grafts may be deployed or installed prior to the deployment or installation of either or both of first and second tubular body portions 12 and 14.

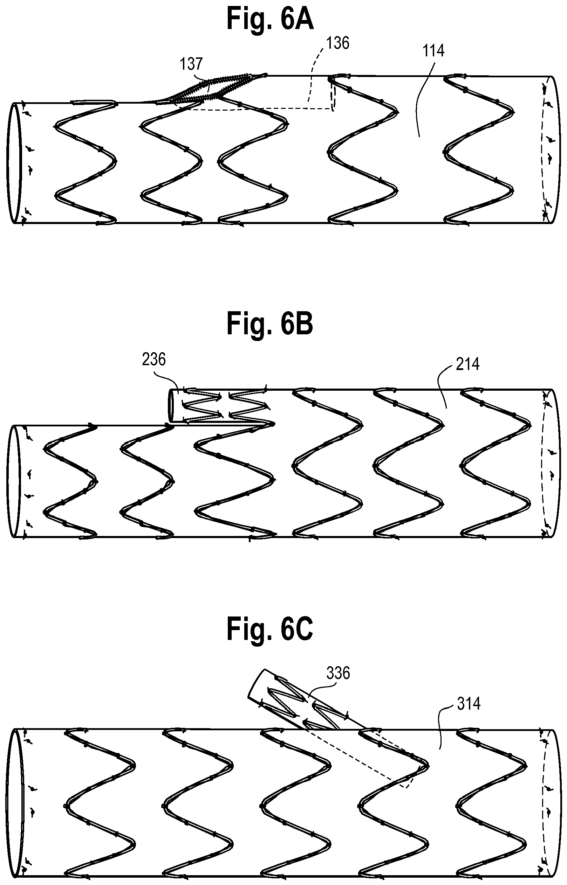

The branch grafts may communicate with the tubular body portions in a variety of ways. For example, as shown in FIG. 6A, second tubular body portion 114 may comprise an internal tube 136 (or internal tube 36 shown in FIG 4) with an opening at its distal and proximal ends and a lumen therethrough for receiving the third branch graft 28. A diamond-shaped opening 137 may further be provided for receiving and directing a branch vessel during installation. In the embodiment of FIG. 6B, second tubular body portion 214 may comprise an external tube 236 for receiving the third branch graft 28. In other embodiments, external tube 236 may communicate directly with a corresponding branch vessel (such as the left subclavian artery), which may eliminate the need for a modular, separate third branch graft altogether. As shown in FIG. 6C, a tube 336 may extend from second tubular body portion 314, and may receive a branch stent graft therein or may communicate directly with a branch vessel without the use of a branch stent graft. However, a modular system is not limited to any of the embodiments and may use any suitable structure and method for a tubular body with a branch stent graft extending therefrom.

Advantageously, the embodiments described herein may eliminate the need for a surgical transposition or a bypass from one branch vessel to another. For example, as depicted in FIG. 2, each of branch grafts 24, 26, and 28 communicates directly with respective arteries 96, 98, and 100. Accordingly, each of the arteries is in fluid communication with the thoracic arch. This is accomplished without a surgical transposition commonly used in the prior art, for example providing communication between carotid artery 98 and left subclavian artery 100. This may avoid highly-invasive surgical techniques and substantial modifications of a patient's natural anatomy.

Many modifications and other embodiments of the invention will come to the mind of one skilled in the art having the benefit of the teachings presented in the foregoing descriptions and associated drawings. Therefore, it is understood that the invention is not to be limited to the specific embodiments disclosed, and that modifications and embodiments are intended to be included within the scope of the appended claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 14/871,394, from which this application claims priority, and in the abstract accompanying this application are incorporated herein by reference.

## Claims

1. A modular system for placement in a patient, the modular system comprising:
a first tubular body portion and a second tubular body portion, wherein the second tubular body portion is located at least partially distally of the first body portion, and wherein the first body portion and the second body portion are engaged at a junction,
wherein a distal end of the first tubular body portion comprises a first taper, wherein a proximal end of the second tubular body portion comprises a second taper, and wherein the first and second tapers form a working space in a deployed state;
a proximal branch graft coupled to the first tubular body portion and extending through a first portion of the working space in the deployed state; and
a distal branch graft coupled to the second tubular body portion and extending through a second portion of the working space in the deployed state.

2. The modular system of claim 1, wherein the proximal branch graft comprises a proximal opening facing proximally within the first tubular body portion.

3. The modular system of claim 1 or 2 further comprising a third tubular body portion located distally of the second tubular body portion and coupled to the second tubular body portion at a second junction.

4. The modular system of any preceding claim, wherein the proximal branch graft is a first branch graft, wherein a second branch graft is coupled to one of the first tubular body portion or the second tubular body portion, and wherein the distal branch graft is a third branch graft.

5. The modular system of claim 4, wherein the second branch graft is coupled to the first tubular body portion and extends through a portion of the working space in the deployed state.

6. The modular system of claim 4 or 5, wherein the first branch graft comprises a first opening disposed within the first tubular body portion, wherein the second branch graft comprises a second opening disposed within the first tubular body portion, and wherein the third branch graft comprises third opening disposed within the second tubular body portion.

7. The modular system of claim 6, wherein the first and second openings face a first direction within the first tubular body portion, and wherein the third opening faces a second direction within the second tubular body portion.

8. The modular system of claim 7, wherein the first direction is proximally, and wherein the second direction is distally.

9. A modular system for placement in a patient, the modular system comprising:
a first tubular body portion and a second tubular body portion, wherein the first tubular body portion at least partially overlaps with the second tubular body portion;
a first branch graft coupled to the first tubular body portion, the first branch graft having a first opening disposed within the first tubular body portion;
a second branch graft coupled to the first tubular body portion, the second branch graft having a second opening disposed within the first tubular body portion; and
a third branch graft coupled to the second tubular body portion, the third branch graft having a third opening disposed within the second tubular body portion,
wherein the first and second openings face a first direction within the first tubular body portion, and wherein the third opening faces a second direction within the second tubular body portion.

10. The modular system of claim 9, wherein a proximal end of the second tubular body portion overlaps a distal end of the first tubular body portion.

11. The modular system of claim 9 or 10 further comprising a third tubular body portion located at least partially distally of the second tubular body portion.

12. The modular system of any of claims 9 to 11, wherein the first direction is proximally, and wherein the second direction is distally.

13. The modular system of any of claims 9 to 12, wherein a proximal end of the second tubular body portion comprises a taper.

14. The modular system of any of claims 9 to 13, wherein a distal end of the first tubular body portion comprises a first taper, wherein a proximal end of the second tubular body portion comprises a second taper, and wherein the first and second tapers form a working space.

15. The modular system of claim 14, wherein the first branch graft is configured to be deployed through the working space.
